# EUROPEAN PATENT APPLICATION

(11) **EP 1 138 282 A1**
(43) Date of publication of application: **04.10.2001**
(21) Application number: 01108076.9
(22) Date of filing: 29.03.2001
(51) Int. Cl.: A61F 2/16

(54) **Intraocular lens assembly**

(30) Priority: 30.03.2000 US 538910
(71) Applicant: Kelman, Charles D., M.D., New York New York 10022 (US)
(72) Inventor: Kelman, Charles D., M.D., New York New York 10022 (US)
(74) Representative: Spott, Gottfried, Dr.

(57) **Abstract**

A two-piece intraocular lens assembly to be implanted within an eye through a minimum size incision into the eye includes a lens optic (10) and a frame assembly (20). The lens optic is flexible and includes radially directed tabs (16) extending from its peripheral edge. The frame assembly, according to a first embodiment of the invention, includes a frame and a pair of haptics (30). The frame has a central opening which is defined by an inside peripheral surface and which is sized and shaped to snugly receive the lens optic. Recesses (32) are formed along the inside peripheral surface of the frame and are adapted to snugly receive and engage with the tabs so that engagement of the tabs into the recesses holds the lens optic within the frame. According to a second embodiment of the invention, a continuous circumferential channel (320) is provided along the inside peripheral surface of the frame to accommodate and engage with all tabs of the lens optic.

## Description

### Background of the Invention

### a) Field of the Invention:

The present invention relates to the field of intraocular lenses. More specifically, the present invention relates to a two piece artificial intraocular lens assembly implantable within an eye for replacing or supplementing a natural lens.

### b) Description of the Prior Art:

In the human eye, the natural lens is positioned behind the pupil and iris and functions to focus light entering the eye onto the retina located at the rear of the eye. A healthy lens is a biconvex, highly transparent structure made of slender, curved rod-shaped ectodermal cells in concentric lamellae surounded by a thin capsule. The lens capsule is supported at its periphery by suspensory ligaments called zonules that are continuous with the ciliary muscles. Through controlled contractions of the ciliary muscles, the shape of the lens capsule may be altered, thereby changing the effective focal length of the lens and aiding in sharply focusing the incoming image light onto the retina.

A cataract condition of the lens results when the material within the lens capsule becomes clouded, thereby obstructing the passage of the incoming image light. To correct this condition, the natural lens is removed by surgery, typically by either intracapsular cataract extraction wherein the entire lens is removed intact, or by extracapsular cataract extraction wherein the hardened lens is disintegrated within the lens capsule using appropriate vibrating cutters and simultaneously removed from the eye using a suction irrigator. Once removed, the damaged natural lens is replaced with an artificial intraocular lens (IOL).

To minimize trauma and the possibility of injury to the eye, the damaged natural lens is typically removed from the eye through a small incision located in the wall of the cornea. It is through this incision that the replacement lens will be inserted, positioned, and secured within the anterior or posterior chamber of the eye.

The IOL is implanted in an anterior or posterior chamber of an eye following the removal of the damaged natural lens. A typical IOL includes a lens optic which is positioned within the eye in the same position as the natural lens so that the lens optic sharply focuses light entering the eye onto the retina. The lens optic is held in position using one or more haptics which are flexible or semi-rigid arm-like structures which extend outwardly from a periphery of the lens optic and contact a portion of the anterior or posterior chamber wall. Conventional haptics are available in any of a variety of different configurations including the J-loop type and the C-loop type. U.S. Patents Nos. 4,174,543 and 5,071,432 disclose examples of haptics. The subject matter of both of these patents is hereby incorporated by reference in its entirety. Regardless of the particular shape, the haptics are preferably made from a highly resilient material so that after being deformed during implantation in the eye they quickly regain their original shape and support the lens optic in position between the cornea and retina.

The haptics of conventional IOLs are usually secured directly to the lens optic, such as by adhering one end of each haptic into a pre-formed bore located along the perimeter of the lens optic. In some instances, the haptics may be integrally formed with the lens optic, and it is also known to provide a frame structure onto which a lens optic may be mounted after both parts are first separately inserted into the eye. In this latter instance, the haptics are secured to the frame structure, instead of to the lens optic.

A two-piece intraocular lens arrangement allows the frame assembly and the attached haptics to collapse together to be inserted within the small corneal incision during the implanting procedure. After the frame assembly and haptics are secured in a proper position within the eye, the flexible lens optic may be similarly collapsed and inserted within the same small incision. Once within the eye, the lens optic may be mounted to the frame assembly.

By separating the lens optic from the frame assembly prior to insertion, the surgeon may easily position the frame assembly within the posterior or anterior chambers of the eye without fear of mechanically abrading or otherwise damaging the delicate lens optic. The lens optic, once inserted into the eye, is secured to the frame assembly.

U.S. Patent. No. 4,833,890 discloses a two piece intraocular lens including an elastic frame having an opening defined by an inner peripheral edge. The inner-peripheral edge includes a securing channel. Two opposing flexible haptics are secured to the frame and are used to secure the entire assembly within the eye. A lens optic, shaped similar to the opening, is made from a rigid optic material and includes a peripheral edge which is sized and shaped to snugly fit within the inner peripheral channel of the frame. The rigid lens is not inserted into the peripheral edge of the frame, rather the frame is flexed (enlarged) around the peripheral edge of the lens and allowed to contract (due to its own resiliency) around the lens, thereby securing the lens in place.

US Patent. No. 4,596,578 discloses a two-piece intraocular lens assembly similar to the one disclosed in US 4,833,890, discussed above, including a flexible frame having an opening with a receiving channel, haptics secured to the frame, and a rigid lens optic that includes a peripheral edge which is sized and shaped to snugly fit within the receiving channel. In this case, however, the frame assembly is split so that it may be selectively "opened" against the spring bias of the frame material. Once opened, the receiving channels of the frame are accessible for receiving the periphery of the lens optic.

US Patent No. 5,074,876 discloses a two-piece intraocular lens which includes an elastic frame having an opening defined by an inner peripheral edge and two opposing haptics secured to the frame. As in the intraocular lenses disclosed in US 4,833,890 and US 4,596,578, a rigid lens optic is secured within the opening of the elastic frame. The rigid lens includes a peripheral groove which is sized to receive an inside edge of the opening of the frame. As in the above-described prior art, the frame of US 5,074,876 is elastically deformed to fit around the peripheral edge of the rigid lens optic.

In each of the above-described two-piece intraocular lenses of the prior art, after the rigid lens optic is passed through the corneal incision and positioned within the eye, the surgeon must apply considerably force and dexterity to elastically deform the elastic frame around the peripheral edge of the lens optic to snugly seat the lens optic within the opening of the frame assembly. This relatively simple procedure proves to be quite difficult because the inherent resilient characteristics of the frame tends to cause a seated edge of the lens to pop free while attempting to deform the elastic frame around the opposing side of the rigid lens. To overcome this unstable lens movement during the seating of the lens, the surgeon must hold both sides of the elastic frame as it is stretched and deformed around the peripheral edge of the lens. This two-point deforming action required to seat a frame around a rigid lens optic is difficult to perform using surgical instruments that are positioned within a small space.

It would be desirable to provide a two-piece minimum size intraocular lens for easy implantation into and assembly in an eye, following removal of the natural lens. Ideally, the lens components could be inserted into the eye using the same minimum size corneal incision used to remove the natural lens. The lens components should be simple in structure and easy to assemble in the relatively small available space within the eye. The components should also be easy to fabricate at relatively low cost from a wide variety of materials.

A first object of the invention is to provide a two-piece artificial intraocular lens which overcomes the drawbacks and deficiencies of the prior art

Another object of the invention is to provide a two-piece artificial intraocular lens which includes a frame assembly and a lens optic that is easily seated within the frame assembly previously implanted in an eye.

Another object of the invention is to provide a two-piece artificial intraocular lens which is inexpensive to fabricate and assemble.

### Summary of the Invention

A two-piece intraocular lens assembly to be implanted within an eye through a minimum size incision into the eye includes a lens optic and a frame assembly. The lens optic according to a first embodiment has radially directed tabs extending from its peripheral edge. The frame assembly, according to a first embodiment of the invention, includes a frame and a pair of haptics. The frame has a central opening which is defined by an inside peripheral surface and which is sized and shaped to snugly receive the lens optic. Recesses are formed along the inside peripheral surface of the frame and are adapted to snugly receive and engage with the tabs so that engagement of the tabs into the recesses holds the lens optic within the frame. According to a second embodiment of the invention, a continuous circumferential channel is provided along the inside peripheral surface of the frame to accommodate and engage with all tabs of the lens optic or with a continuous circumferential bead formed on the outer peripheral surface of the lens optic.

According to the invention, a method for assembling a flexible intraocular lens optic having a circumferential bead within a frame assembly having a inside circumferential channel, includes the steps of deforming the flexible lens optic and seating a portion of the circumferential bead of the lens within the channel of the frame. Continuing to deform the unseated portions of the lens optic until the entire periphery of the lens optic is seated within the channel of the frame.

According to the invention, a further method for assembling the intraocular lens optic having at least two tabs projecting from the peripheral edge within a frame assembly having at least two recesses each of which being sized to accommodate each of said tabs, includes the steps of deforming the flexible lens optic to seat one tab into one recess of the frame. Continuing to deform the unseated portions of the lens optic until the remaining tabs are seated within their respective recesses and the entire periphery of the lens optic is seated within the frame.

### Brief Description of the Drawings

Fig. 1 is a front view of an intraocular lens optic, according to a first embodiment of the present invention;
Fig. 2 is a side view of the intraocular lens optic of Fig. 1, according to the first embodiment of the present invention;
Fig. 3 is a front view of an empty frame assembly according to the first embodiment of the present invention, including a frame, two opposing, integrally formed haptics, and spaced recesses located within an inside peripheral surface;
Fig. 4 is a partial sectional view of the empty frame of Fig. 3, according to the first embodiment of the present invention, taken along the lines 4-4 of Fig. 3;
Fig. 5 is a front view of a frame assembly shown supporting a lens optic within the frame, according to the first embodiment of the invention;
Fig. 6 is a partial sectional view of the frame assembly of Fig. 5, according to the second embodiment of the present invention, taken along the lines 6-6 of Fig. 5;
Fig. 7 is a front view of an empty frame assembly according to a second embodiment of the present invention, including a frame, two opposing, integrally formed haptics and an inside peripheral channel; and
Fig. 8 is a partial sectional view of the frame assembly according to the second embodiment of the present invention, taken along the lines 8-8 of Fig. 7.

### Detailed Description of the Preferred Embodiments

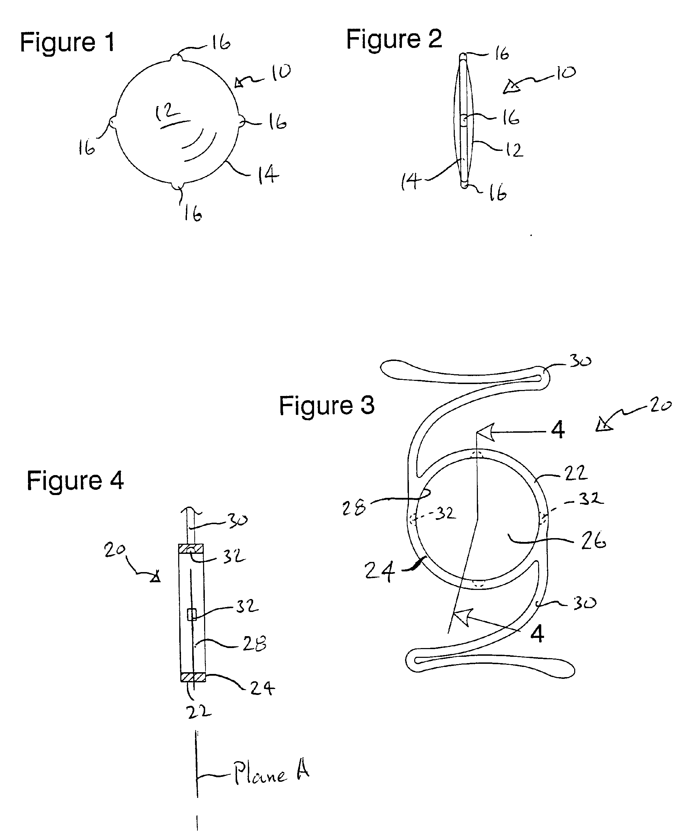

Referring to Figs. 1 and 2, a lens optic 10 according to a first embodiment is shown including a central magnifying portion 12, a peripheral edge 14, and preferably four evenly spaced tabs 16. Tabs 16 and central magnifying portion 12 are preferably formed integrally during the manufacture of lens optic 10. As an alternative, tabs 16 may also be formed separate from central magnifying portion 12 and later secured at evenly spaced locations along peripheral edge 14 using conventional adhesives or appropriate bonding techniques.

Lens optic 10 (including tabs 16 and central magnifying portion 12) is generally circular and preferably made from flexible (or otherwise deformable) material, such as hydrogel-based material, silicone material, foldable acrylic, or the like.

A frame assembly 20, according to the first embodiment of the invention is shown in Figs. 3 and 4. Frame assembly 20 includes a generally circular frame 22 having a circular front face 24 and a central opening 26 which is defined by an inside peripheral surface 28. Frame assembly 20 preferably further includes two integrally formed opposing haptics (or fixation elements) 30 which are sized and shaped to engage the contours of the anterior or posterior chambers of the eye (depending on the type of surgical procedure being performed and the particular condition being corrected) to effectively support the entire intraocular lens assembly (i.e., lens optic 10 and frame assembly 20) in a proper position. Frame assembly 20 may be made from a variety of flexible, semi-rigid, or rigid materials, such as a single piece of polymethylmethacrylate (PMMA), or any other appropriate biologically inert plastic material. Alternatively, frame 22 and haptics 30 may be formed separately and later connected by adhesive bonding or ultrasonic welding or any other connection method known in the art. The cross sectional shape, diameter and length of the haptics 30 as well as the angles at which they project from frame 22 and the degree of curvature of the legs of haptics 30 all depend on a variety of factors and conditions which are well known to those skilled in the art and are beyond the scope of the present invention.

Although a two-haptic arrangement is illustrated in the Figures of the present invention, other haptic arrangements may be used as desired.

Located along the inside peripheral surface 28 and formed within frame 22 are preferably four evenly spaced recesses (or slots) 32. Each recess 32 is sized and shaped to accommodate one tab 16 of lens optic 10. All four recesses 32 are preferably evenly spaced around the inside peripheral surface 28 and are all preferably coplanar, i.e., all lie within the same plane, as shown in Fig. 4, which is preferably parallel to frame 22 and haptics 30. Recesses 32 are preferably formed during the manufacture of frame assembly 20 using conventional molding techniques well known in the art.

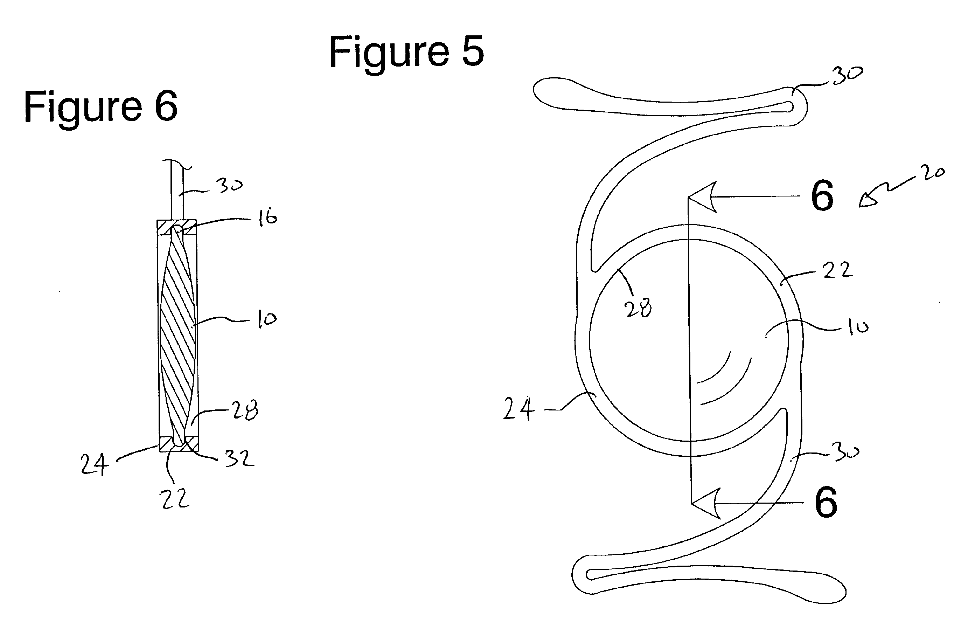

Lens optic 10 is sized and shaped to snugly fit within central opening 26 of frame 22, that is the outer diameter of central magnifying portion is preferably equal to or slightly smaller than the inner diameter of central opening 26. Tabs 16 along the peripheral edge 14 of lens optic 10 are spaced in alignment with recesses 32 so that when lens optic 10 is positioned within circular opening 26 of frame assembly 20 and displaced along inside peripheral surface 28 to plane A (See Fig. 4), tabs 16 may align and engage with recesses 32, one recess 32 for each tab 16. The resiliency of the hydrogel material used to make lens optic 10 and integrally formed tabs 16 allows sufficient flexure of tabs 16 and the lens optic 10 to deform during the positioning of lens optic 10 with respect to frame 22 as lens optic 10 is being seated within central opening 26 of frame assembly 20. Once a tab 16 aligns with a recess 32, its natural resiliency will cause tab 16 to spring into engagement within recess 32 of frame 22. Lens optic 10 will become secured within frame assembly 20 (within plane A) when all four tabs 16 are engaged within their corresponding recesses 32, as shown in Figs. 5 and 6.

According to this first embodiment of the invention, once peripheral edge 14 of lens optic 10 (and therefore tabs 16) are within plane A, it may be necessary to rotate lens optic 10 with respect to frame assembly 20 until each of the four tabs 16 align and engage with each of the four recesses 32.

As discussed above in the Background of the Invention section of the present patent application, one problem encountered when surgically implanting two-piece type intraocular lens assemblies within an eye involves the difficulty of "snapping" the lens optic 10 into frame 22. The present invention eliminates this problem by providing isolated tabs 16, according to one embodiment, which may flex independent of each other so that engagement of one tab 16 within a recess 32 of frame 22 will not affect the positioning and engagement of any of the remaining three tabs 16 into their respective recesses 32. Preferably, one peripheral edge portion of the lens optic is placed within the central opening 26 adjacent the corresponding portion of inside peripheral surface 28 (placing two tabs 16 into the corresponding recesses 32). The optic is then flexed slightly so as to insert the opposite pair of tabs 16 into the opposite pair of recesses 32 in response to the optic 10 being allowed to resume its undeformed state once again.

As is well known in the art, a problem associated with prior art intraocular lenses is that a glare effect may be perceived by the patient under certain circumstances such as when the patient's pupil is fully dilated. In such instance, light entering the eye may be able to pass along the periphery of the implanted intraocular lens and create the illusion of glare. To overcome this problem, the frame 22 of the present invention functions as a shield against glare by blocking or at least changing the direction of light rays trying to pass adjacent to the periphery of lens optic 10. Front surface 24 of frame 22 may be scored or otherwise coated or altered to effectively block any impinging light in this region. The use of a frame to help prevent glare is disclosed in U.S. Patent No. 5,769,889 which is hereby incorporated by reference, in its entirety.

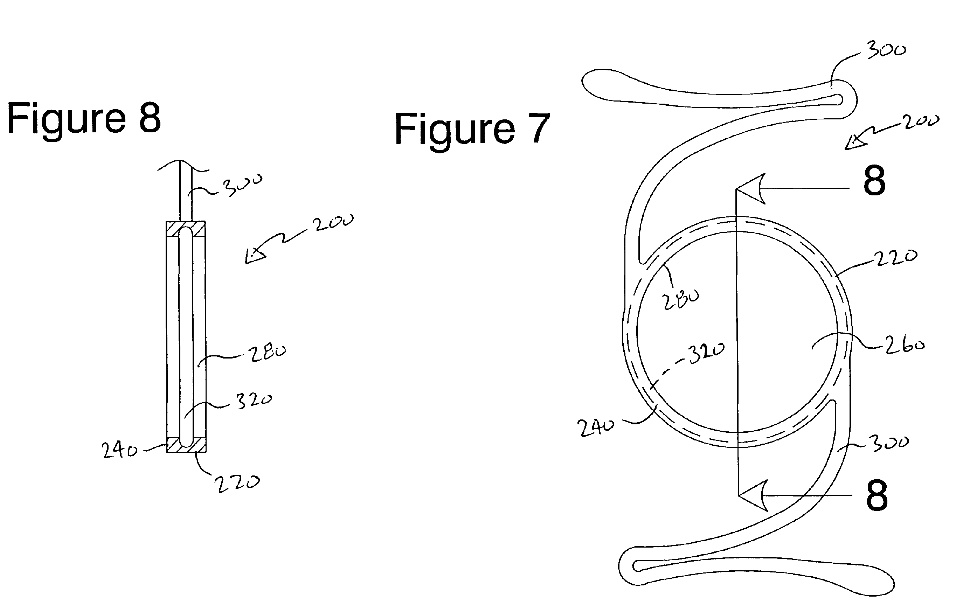

Referring now to Figs. 7 and 8, a second embodiment of the present invention is shown including a frame assembly 200 having integrally formed haptics 30 (two opposing haptics are shown), and a frame 220. Frame 220, as in the above described first embodiment includes a central opening 260 which is defined by an inside peripheral surface 280 and includes a front face 240. According to this second embodiment of the invention, a continuous circumferential groove or channel 320 is formed within inside peripheral surface 280. Channel 320, which defines a plane B, as shown in Fig. 8, replaces the four recesses 32 of the above-described first embodiment of the invention. According to this second embodiment, the lens optic remains unchanged with four (or any number) tabs 16 evenly spaced and projecting radially from the peripheral edge 14 (See Figs. 1 and 2).

In use of the frame assembly 200, according to this second embodiment, after frame assembly 200 is inserted through a small corneal incision (not shown) and positioned and secured within an anterior or posterior chamber of the eye, lens optic 10 may be inserted within the same corneal incision and secured within central opening 260 of frame 220. Lens optic 10 may be seated within frame 220 in a similar manner as described above for seating lens optic 10 into frame 22 according to the first embodiment of the invention, however, the improvement of the frame assembly 200 of the second embodiment obviates the requirement to rotationally align the tabs 16 with their matching recesses 32. According to this second embodiment, as lens optic 10 is inserted within central opening 260 of frame 220, once any tab 16 reaches plane B, the tab 16 will automatically extend into and engage with channel 320 regardless of the relatively angular orientation between lens optic 10 and frame assembly 200.

In either of the above described embodiments, tabs 16 of lens optic 10 effectively reduce the difficulties of attaching a lens optic 10 with its frame assembly 20 (or 200).

While the invention has been particularly shown and described with reference to a preferred embodiment thereof, it will be understood by those skilled in the art that various changes in form and details may be made herein without departing from the spirit and scope of the invention.

## Claims

1. A two-piece intraocular lens assembly to be implanted within an eye through a minimum size incision into the eye; comprising:
a lens optic having a central optic portion, a peripheral edge, and at least two tabs extending radially outward from said peripheral edge; and
a frame assembly having a frame and at least one haptic attached to said frame and extending therefrom, said frame having an opening defined by an inside peripheral surface, said opening being sized and shaped to receive said lens optic, said inside peripheral surface of said frame including at least two recesses which are sized and shaped to selectively receive and engage with said at least two tabs of said lens optic to hold said lens optic within said frame of said frame assembly.

2. The lens assembly according to claim 1, wherein said frame is optically opaque.

3. The lens assembly according to claim 1, wherein said frame is optically translucent.

4. The lens assembly according to claim 1, wherein said frame assembly is comprised of PMMA material.

5. The lens assembly according to claim 1, wherein said lens optic is made from a hydrogel material.

6. The lens assembly according to claim 1, wherein said tabs are integrally formed with said lens optic;

7. The lens assembly according to claim 1, wherein said tabs are formed separate from said lens optic and attached to said peripheral edge of said lens optic using an adhesive.

8. The lens assembly according to claim 7, wherein said tabs are attached to said peripheral edge of said lens optic using ultrasonic bonding.

9. The lens assembly according to claim 1, wherein said lens optic includes four tabs evenly spaced about said peripheral edge, and said frame includes four evenly spaced recesses which are positioned to align with said tabs.

10. A two-piece intraocular lens assembly to be implanted within an eye through a minimum size incision into the eye; comprising:
a generally circular lens optic having a central optic portion, a peripheral edge, and at least two tabs extending radially outward from said peripheral edge; and
a frame assembly having a frame and at least one haptic, said at least one haptic being attached to said frame and extending therefrom, said frame having an opening which is defined by an inside peripheral surface, said opening being sized and shaped to receive said lens optic, said inside peripheral surface of said frame including a circumferential channel which is sized and shaped to snugly receive and engage with said tabs of said lens optic so that said tabs engaged with said channel and hold said lens optic within said frame of said frame assembly.

11. The lens assembly according to claim 10, wherein said frame is optically opaque.

12. The lens assembly according to claim 10, wherein said frame is optically translucent.

13. The lens assembly according to claim 10, wherein said frame assembly is comprised of PMMA material.

14. The lens assembly according to claim 10, wherein said lens optic is made from a hydrogel material.

15. The lens assembly according to claim 10, wherein said tabs are integrally formed with said lens optic.

16. The lens assembly according to claim 10, wherein said tabs are formed separate from said lens optic and attached to said peripheral edge of said lens optic using an adhesive.

17. The lens assembly according to claim 16, wherein said tabs are attached to said peripheral edge of said lens optic using ultrasonic bonding.

18. The lens assembly according to claim 10, wherein said lens optic includes four tabs evenly spaced about said peripheral edge, and said frame includes four evenly spaced recesses which are positioned to align with said tabs.

19. The lens assembly according to claim 10, wherein said lens optic is made from silicone material.

20. The lens assembly according to claim 10, wherein said lens optic is made from a foldable acrylic material.

21. A method for securing a flexible lens optic within a frame assembly, said lens optic having a peripheral edge, said frame assembly including an opening having an inside peripheral groove, said groove being sized to receive said peripheral edge, said securing method comprising:
seating a portion of said peripheral edge of said lens optic within said peripheral groove of said frame assembly;
deforming said flexible lens optic; and
seating the remaining peripheral edge portions of said lens optic within said peripheral groove of said frame assembly.
